# EUROPEAN PATENT SPECIFICATION

(11) **EP 3 548 022 B1**
(45) Date of publication and mention of the grant of the patent: **22.11.2023**
(21) Application number: 17877189.5
(22) Date of filing: 01.12.2017
(51) Int. Cl.: A61K 31/4184, C07C 225/26, C07C 225/34, C07D 235/02, A61K 31/122, A61K 31/167, A61K 31/17, A61K 31/196, A61K 31/366, A61K 31/4192, A61K 31/421, A61K 31/4709, A61K 31/4725, A61K 31/473, A61K 31/4745, A61K 31/506, A61K 31/53, A61P 31/04, A61P 35/00

(54) **COMPOSITIONS FOR MODULATING PD-1 SIGNAL TRANSDUCTION**
ZUSAMMENSETZUNGEN ZUR MODULIERUNG DER PD-1-SIGNALTRANSDUKTION
COMPOSITIONS POUR MODULER LA TRANSDUCTION DU SIGNAL PD-1

(30) Priority: 02.12.2016 US 201662429126 P
(43) Date of publication of application: 09.10.2019
(73) Proprietor: Augusta University Research Institute, Inc., Augusta, GA 30912 (US)
(72) Inventor: KHLEIF, Samir, Silver Springs, Georgia 20906 (US); MKRTICHYAN, Mikayel, Tujunga, California 91042 (US); LEBEDYEVA, Iryna, Augusta, GA 30914-3029 (US); ALBERS, Thomas, Augusta, Georgia 30912 (US)
(74) Representative: J A Kemp LLP
(86) International application number: PCT/IB2017/057586
(87) International publication number: WO 2018/100556

(56) References cited:
- WO-A1-2018/038886
- WO-A1-2019/050890
- US-A1- 2013 022 629
- US-A1- 2015 073 024

## Description

### FIELD OF THE INVENTION

The invention is generally directed to pharmaceutical compositions, wherein said pharmaceutical compositions may be for use in a method of modulating immune responses in a subject in need thereof.

### BACKGROUND

The response of T lymphocytes to disease states, such as infection and chronic diseases like cancer, is complicated and involves intercellular interactions and the production of soluble mediators (called cytokines or lymphokines). Activation of T cells normally depends on an antigen-specific signal following contact of the T cell receptor (TCR) with an antigenic peptide presented via the major histocompatibility complex (MHC) while the extent of this reaction is controlled by positive and negative antigen-independent signals emanating from a variety of co-stimulatory molecules. The latter are commonly members of the CD28/B7 family. Conversely, Programmed Death-1 (PD-1) is a member of the CD28 family of receptors that delivers a negative immune response when induced on T cells. Contact between PD-1 and one of its ligands (PD-L1 or PD-L2) induces an inhibitory response that decreases T cell multiplication and/or the strength and/or duration of a T cell response.

The PD-1/PD-1 ligand (PD-L) interaction is one of the significant mechanisms that tumors use to inhibit effector T cells both in periphery and within tumor microenvironment. The primary result of PD-1 ligation by its ligands is to inhibit signaling downstream of the T cell Receptor (TCR). Therefore, signal transduction via PD-1 usually provides a suppressive or inhibitory signal to the T cell that results in decreased T cell proliferation or other reduction in T cell activation. PD-L1 is the predominant PD-1 ligand causing inhibitory signal transduction in T cells. To date several anti-PD-1 and anti-PD-L1, anti-PD-L2 antibodies, and ECD-Fc fusion proteins that block PD-1/PD-L interactions are developed, in clinic or already approved by Food and Drug Administration. Blocking of this inhibitory interaction is shown to be potent both in pre-clinical and clinical studies.

Negative signal transduction may not be an only transmitted signal from PD-1 and potentially some activating signal may be transmitted with the proper engagement site.

Therefore, it is an object of the invention to provide compositions and methods for inducing T cell activation.

It is another object of the invention to provide compositions and methods for inducing or promoting a T cell activating signal through PD-1.

It is still another object of the invention to provide treatment regimens for treating diseases through increased T cell activity, especially cancer and infectious diseases.

US2013/022629 relates to an assay for identifying modulators of the PD-1:PD-L pathway and PD-1:PD-L pathway modulators.

US2015/0073024 relates to 1,2,4-oxadiazole and 1,2,4,-thiadiazole compounds as therapeutic agents capable of inhibiting the programmed cell death (PD1) signaling pathway.

### SUMMARY

Several compounds have been discovered that engage with PD-1 receptor and send an activating signal (induction of IFNgamma production) in T cells. One embodiment provides a pharmaceutical composition comprising (a) one or more PD-1 modulating compounds selected from the group consisting of 1-(1H-benzo[d][1,2,3]triazol-1-yl)anthracene-9,10-dione; 1,1'-(oxybis(4,1-phenylene))bis(3-(2-chlorophenyl)urea); 5,5'-diphenyl-2,2',3,3'-tetrahydro-2,2'-bibenzo[d]oxazole; 2-(isoquinolin-1-yl)-5-phenyl-4-(p-tolyl)oxazole; 2'-((6-oxo-5,6-dihydrophenanthridin-3-yl)carbamoyl)-[1,1'-biphenyl]-2-carboxylic acid; 2'-((6-oxo-6H-benzo[c]chromen-2-yl)carbamoyl)-[1,1'-biphenyl]-2-carboxylic acid; 3-(4-chloro-6-phenoxy-1,3,5-triazin-2-yl)-1-phenyl-1H-indole; 1,8-bis(phenylthio)anthracene-9,10-dione; 4-chloro-2-(3-(phenylthio)phenyl)quinoline-6-sulfonyl fluoride; bis(2,2,4-trimethyl-1,2-dihydroquinolin-6-yl)methane; 2-nitro-4-((6-nitroquinolin-4-yl)amino)-N-(4-(pyridin-4-ylamino)phenyl)benzamide; 1,2-bis(4-isopropyl-6-(trifluoromethyl)pyrimidin-2-yl)hydrazine; 3-(benzylthio)phenanthro[9,10-e][1,2,4]triazine; (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9, 10, 11, 12, 12a, 12b, 13, 14b-octadecahydropicene-2-carbonitrile (CDDO-Im); 2-(1H-phenanthro[9,10-d]imidazol-2-yl)phenol; 3-(4,5-dimethylbenzo[h][1,6]naphthyridin-2-yl)-2-methylquinolin-4-amine; N-(4-bromonaphthalen-1-yl)-1-hydroxy-2-naphthamide; N-(3-(pyridin-2-yl)isoquinolin-1-yl)picolinimidamide; 2-(isoquinolin-1-yl)-4,5-diphenyloxazole; 2,2'-((3,3'-dimethoxy-[1,1'-biphenyl]-4,4'-diyl)bis(azanediyl))dibenzoic acid; or solvate, or pharmaceutically acceptable salt thereof; and (b) a pharmaceutically acceptable carrier. These compounds also referred to as PD-1 modulating compounds and compositions bind to specific domain of the PD-1 receptor and either block and/or initiate the activating signaling in T cells. Thus, engaging this site of PD-1 receptor with the disclosed compounds can either block PD-1/PD-L1 interaction and/or can stimulate T cells. The disclosed compounds and compositions are useful for the treatment of immunological disorders and cancers since they either stimulate T cells through otherwise inhibitory receptor and/or block the inhibitory interaction, thus, overcoming immune tolerance associated with PD-1/PD-L pathway.

The current method used for anti-PD1 is to block its negative signal towards T cell, then the T cell requires another signal to become activated. In certain embodiments the disclosed PD-1 modulating compounds are not only 1) preventing the negative signal but also 2) providing an activating signal at the same time.

The one or more PD-1 binding compounds may bind to PD-1 under physiological conditions and promote or induce an activating signal through PD-1 that activates a T cell expressing PD-1. The one or more PD-1 binding compounds may bind to PD-1 receptor under physiological conditions and inhibit, reduce or prevent ligands of PD-1 from binding to PD-1 and thereby inhibit, reduce or prevent negative signal transduction through PD-1 receptor.

The pharmaceutical compositions as described herein may induce or promote T cell activation by increasing an antigen-specific proliferation of T cells, increasing or enhancing cytokine production by T cells, stimulation of differentiation and effector functions of T cells and/or promoting T cell survival) or overcoming T cell exhaustion and/or anergy.

The pharmaceutical compositions as described herein may also induce or promote an immune response in a subject in need thereof, by inducing or promoting T cell activation.

An embodiment provides a pharmaceutical composition for use in a method for treating cancer in a subject, the method comprising administering to the subject an effective amount of the composition to induce or promote T cell activation. The cancer can be bladder, brain, breast, cervical, colo-rectal, esophageal, kidney, liver, lung, nasopharangeal, pancreatic, prostate, skin, stomach, uterine, ovarian, testicular, hematologic, a melanoma, a renal cancer, a myeloma, a thyroid cancer, a lymphoma, a leukemia, or a metastatic lesion of the cancer.

One embodiment provides a pharmaceutical composition for use in a method for treating an infection in a subject in need thereof, the method comprising administering to the subject an effective amount of the composition to promote or induce T cell activation in the subject. The infection can be a microbial infection, for example a bacterial, fungal, or viral infection. In another embodiment, the infection is a parasitic infection.

### DETAILED DESCRIPTION

### I. Definitions

The term "T cell activating signal" or "activation signal" refers to signal transduction through a receptor on a T cell that induces or promotes activation of the T cell. Activation of the T cell includes, but is not limited to an increase in antigen-specific proliferation of T cells, increased or enhanced cytokine production by T cells, stimulation of differentiation and effector functions of T cells and/or promoting T cell survival or overcoming T cell exhaustion and/or anergy.

The term "T cell inhibitory signal" or "inhibitory signal" or "negative signal transduction" refers to signal transduction through a receptor on a T cell that induces or promotes suppression of T cell activity. An exemplary inhibitory signal is the interaction of PD-L1 with the PD-1 receptor which decreases T cell multiplication and/or the strength and/or duration of a T cell response.

The use of the terms "a", "an", "the" and similar referents in the context of describing the presently claimed invention (especially in the context of the claims) are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other embodiments the values may range in value either above or below the stated value in a range of approx. +/- 5%; in other embodiments the values may range in value either above or below the stated value in a range of approx. +/- 2%; in other embodiments the values may range in value either above or below the stated value in a range of approx. +/- 1%. The preceding ranges are intended to be made clear by context, and no further limitation is implied. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

The acronym "ECD" refers to extracellular domain.

As used herein the term "effective amount" or "therapeutically effective amount" means a dosage sufficient to treat, inhibit, or alleviate one or more symptoms of a disease state being treated or to otherwise provide a desired pharmacologic and/or physiologic effect. The precise dosage will vary according to a variety of factors such as subject-dependent variables (e.g., age, immune system health, etc.), the disease, and the treatment being administered.

The terms "individual", "host", "subject", and "patient" are used interchangeably herein, and refer to a mammal, including, but not limited to, humans, rodents, such as mice and rats, and other laboratory animals.

The term "soluble receptor" refers to the extracellular domain (ECD) of a transmembrane protein involved in signal transduction. For example, soluble PD-1 refers the extracellular domain of the PD-1 receptor. The soluble receptor can be a fragment of the extracellular domain of the transmembrane protein that retains its ability to bind to a ligand of the receptor. Soluble receptors include naturally occurring soluble receptors and synthetic (i.e., not naturally occurring) soluble receptors.

### II. Compositions for Modulating PD-1 Signal Transduction

### A. PD-1 Modulating Compounds

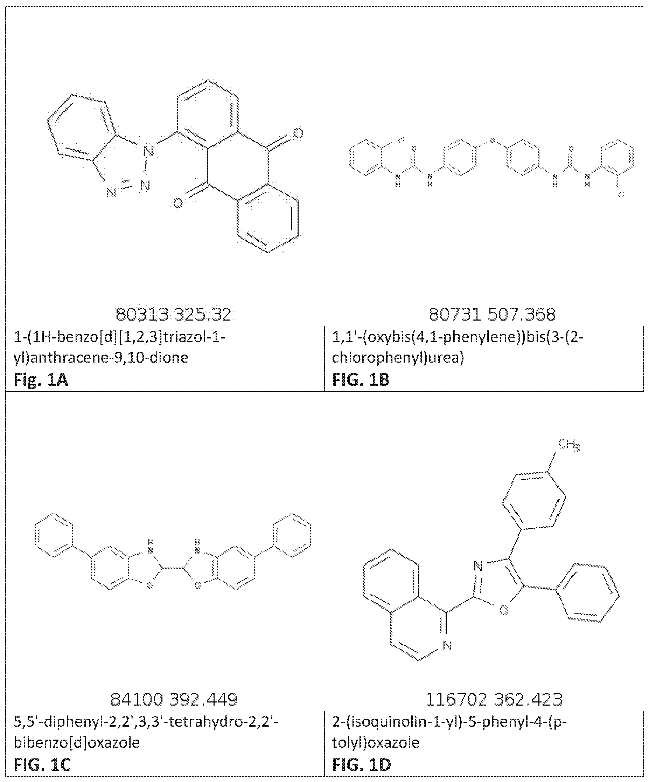

Several compounds have been discovered that modulate signal transduction through the PD-1 receptor. One embodiment provides pharmaceutical formulations for use in modulating PD-1 signal transduction in a subject in need thereof wherein the pharmaceutical formulations contain an effective amount of one or more of the compounds of Figures 1A-1T. In one embodiment, the compounds of Figures 1A-1T bind to PD-1 and promote or induce an activating signal through PD-1 receptor that activates the T cell. In another embodiment, one or more of the compounds of Figures 1A-1T bind to PD-1 and inhibit the binding of ligands, such as PD-L1 and PD-L2, to the PD-1 receptor, and thereby block transduction of a T cell inhibitory signal through the PD-1 receptor. In still another embodiment, the compounds of Fig. 1A-1T bind to PD-1 and block ligands from binding to PD-1 as well as promote or induce an activating signal through PD-1 that activates the T cell.

### 1. 1-(1H-benzo[d][1,2,3]triazol-1-yl)anthracene-9,10-dione

Fig. 1A shows the structure of 1-(1H-benzo[d][1,2,3]triazol-1-yl)anthracene-9,10-dione which binds to PD-1 and modulates signal transduction though the PD-1 receptor. In one embodiment, 1-(1H-benzo[d][1,2,3]triazol-1-yl)anthracene-9,10-dione binds to PD-1 and promotes or induces an activating signal though PD-1 to activate the T cell. In another embodiment, 1-(1H-benzo[d][1,2,3]triazol-1-yl)anthracene-9,10-dione binds to PD-1 and blocks signal transduction though PD-1 by blocking the interaction of ligands of PD-1 with the PD-1 receptor. Exemplary ligands of PD-1 that are blocked by 1-(1H-benzo[d][1,2,3]triazol-1-yl)anthracene-9,10-dione include, but are not limited to PD-L1 and PD-L2. In another embodiment 1-(1H-benzo[d][1,2,3]triazol-1-yl)anthracene-9,10-dione binds to PD-1 and blocks ligands from binding to PD-1 as well as promotes or induces an activating signal through PD-1 that activates the T cell.

### 2. 1,1'-(oxybis(4,1-phenylene))bis(3-(2-chlorophenyl)urea)

Fig. 1B shows the structure of 1,1'-(oxybis(4,1-phenylene))bis(3-(2-chlorophenyl)urea) which bind to PD-1 and modulates signal transduction though PD-1 receptor. In one embodiment, 1,1'-(oxybis(4,1-phenylene))bis(3-(2-chlorophenyl)urea) binds to PD-1 and promotes or induces an activating signal though PD-1 to activate the T cell. In another embodiment, 1,1'-(oxybis(4,1-phenylene))bis(3-(2-chlorophenyl)urea) binds to PD-1 and blocks signal transduction though PD-1 by blocking the interaction of ligands of PD-1 with PD-1 receptor. Exemplary ligands of PD-1 that are blocked by 1,1'-(oxybis(4,1-phenylene))bis(3-(2-chlorophenyl)urea) include, but are not limited to PD-L1 and PD-L2. In another embodiment of 1,1'-(oxybis(4,1-phenylene))bis(3-(2-chlorophenyl)urea) binds to PD-1 and blocks ligands from binding to PD-1 as well as promotes or induces an activating signal through PD-1 that activates the T cell.

### 3. 5,5'-diphenyl-2,2',3,3'-tetrahydro-2,2'-bibenzo[d]oxazole

Fig. 1C shows the structure of 5,5'-diphenyl-2,2',3,3'-tetrahydro-2,2'-bibenzo[d]oxazole which bind to PD-1 and modulates signal transduction though PD-1 receptor. In one embodiment, 5,5'-diphenyl-2,2',3,3'-tetrahydro-2,2'-bibenzo[d]oxazole binds to PD-1 and promotes or induces an activating signal though PD-1 to activate the T cell. In another embodiment, 5,5'-diphenyl-2,2',3,3'-tetrahydro-2,2'-bibenzo[d]oxazole binds to PD-1 and blocks signal transduction though PD-1 by blocking the interaction of ligands of PD-1 with PD-1 receptor. Exemplary ligands of PD-1 that are blocked by 5,5'-diphenyl-2,2',3,3'-tetrahydro-2,2'-bibenzo[d]oxazole include, but are not limited to PD-L1 and PD-L2. In another embodiment of 5,5'-diphenyl-2,2',3,3'-tetrahydro-2,2'-bibenzo[d]oxazole binds to PD-1 and blocks ligands from binding to PD-1 as well as promotes or induces an activating signal through PD-1 that activates the T cell.

### 4. 2-(isoquinolin-1-yl)-5-phenyl-4-(p-tolyl)oxazole

Fig. 1D shows the structure of 2-(isoquinolin-1-yl)-5-phenyl-4-(p-tolyl)oxazole which bind to PD-1 and modulates signal transduction though PD-1 receptor. In one embodiment, 2-(isoquinolin-1-yl)-5-phenyl-4-(p-tolyl)oxazole binds to PD-1 and promotes or induces an activating signal though PD-1 to activate the T cell. In another embodiment, 2-(isoquinolin-1-yl)-5-phenyl-4-(p-tolyl)oxazole binds to PD-1 and blocks signal transduction though PD-1 by blocking the interaction of ligands of PD-1 with PD-1 receptor. Exemplary ligands of PD-1 that are blocked by 2-(isoquinolin-1-yl)-5-phenyl-4-(p-tolyl)oxazole include, but are not limited to PD-L1 and PD-L2. In another embodiment of 2-(isoquinolin-1-yl)-5-phenyl-4-(p-tolyl)oxazole binds to PD-1 and blocks ligands from binding to PD-1 as well as promotes or induces an activating signal through PD-1 that activates the T cell.

### 5. 2'-((6-oxo-5,6-dihydrophenanthridin-3-yl)carbamoyl)-[1,1'-biphenyl]-2-carboxylic acid

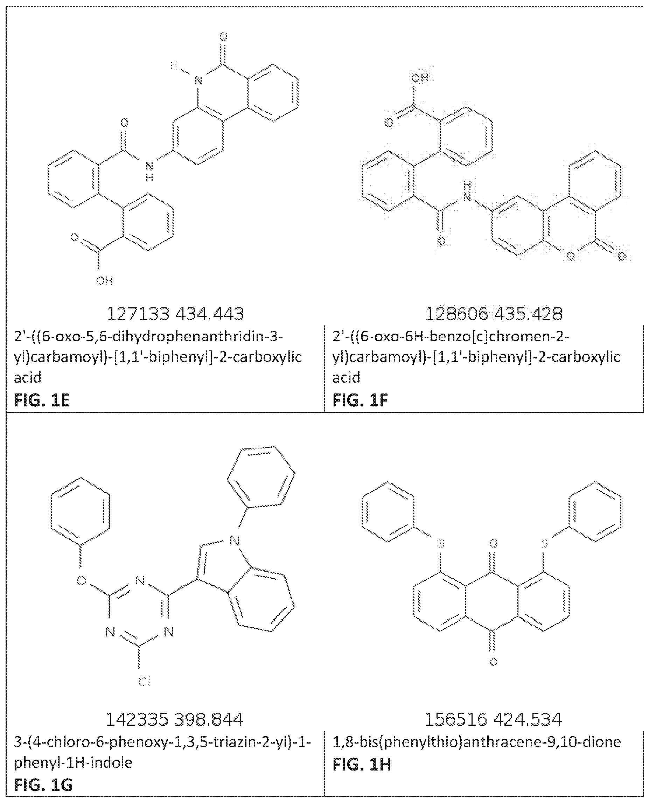

Fig. 1E shows the structure of 2'-((6-oxo-5,6-dihydrophenanthridin-3-yl)carbamoyl)-[1,1'-biphenyl]-2-carboxylic acid which bind to PD-1 and modulates signal transduction though PD-1 receptor. In one embodiment, 2'-((6-oxo-5,6-dihydrophenanthridin-3-yl)carbamoyl)-[1,1'-biphenyl]-2-carboxylic acid binds to PD-1 and promotes or induces an activating signal though PD-1 to activate the T cell. In another embodiment, 2'-((6-oxo-5,6-dihydrophenanthridin-3-yl)carbamoyl)-[1,1'-biphenyl]-2-carboxylic acid binds to PD-1 and blocks signal transduction though PD-1 by blocking the interaction of ligands of PD-1 with PD-1 receptor. Exemplary ligands of PD-1 that are blocked by 2'-((6-oxo-5,6-dihydrophenanthridin-3-yl)carbamoyl)-[1,1'-biphenyl]-2-carboxylic acid include, but are not limited to PD-L1 and PD-L2. In another embodiment of 2'-((6-oxo-5,6-dihydrophenanthridin-3-yl)carbamoyl)-[1,1'-biphenyl]-2-carboxylic acid binds to PD-1 and blocks ligands from binding to PD-1 as well as promotes or induces an activating signal through PD-1 that activates the T cell.

### 6. 2'-((6-oxo-6H-benzo[c]chromen-2-yl)carbamoyl)-[1,1'-biphenyl]-2-carboxylic acid

Fig. 1F shows the structure of 2'-((6-oxo-6H-benzo[c]chromen-2-yl)carbamoyl)-[1,1'-biphenyl]-2-carboxylic acid which bind to PD-1 and modulates signal transduction though PD-1 receptor. In one embodiment, 2'-((6-oxo-6H-benzo[c]chromen-2-yl)carbamoyl)-[1,1'-biphenyl]-2-carboxylic acid binds to PD-1 and promotes or induces an activating signal though PD-1 to activate the T cell. In another embodiment, 2'-((6-oxo-6H-benzo[c]chromen-2-yl)carbamoyl)-[1,1'-biphenyl]-2-carboxylic acid binds to PD-1 and blocks signal transduction though PD-1 by blocking the interaction of ligands of PD-1 with PD-1 receptor. Exemplary ligands of PD-1 that are blocked by 2'-((6-oxo-6H-benzo[c]chromen-2-yl)carbamoyl)-[1,1'-biphenyl]-2-carboxylic acid include, but are not limited to PD-L1 and PD-L2. In another embodiment of 2'-((6-oxo-6H-benzo[c]chromen-2-yl)carbamoyl)-[1,1'-biphenyl]-2-carboxylic acid binds to PD-1 and blocks ligands from binding to PD-1 as well as promotes or induces an activating signal through PD-1 that activates the T cell.

### 7. 3-(4-chloro-6-phenoxy-1,3,5-triazin-2-yl)-1-phenyl-1H-indole

Fig. 1G shows the structure of 3-(4-chloro-6-phenoxy-1,3,5-triazin-2-yl)-1-phenyl-1H-indole which bind to PD-1 and modulates signal transduction though PD-1 receptor. In one embodiment, 3-(4-chloro-6-phenoxy-1,3,5-triazin-2-yl)-1-phenyl-1H-indole binds to PD-1 and promotes or induces an activating signal though PD-1 to activate the T cell. In another embodiment, 3-(4-chloro-6-phenoxy-1,3,5-triazin-2-yl)-1-phenyl-1H-indole binds to PD-1 and blocks signal transduction though PD-1 by blocking the interaction of ligands of PD-1 with PD-1 receptor. Exemplary ligands of PD-1 that are blocked by 2'-((6-oxo-6H-benzo[c]chromen-2-yl)carbamoyl)-[1,1'-biphenyl]-2-carboxylic acid include, but are not limited to PD-L1 and PD-L2. In another embodiment of 2'-((6-oxo-6H-benzo[c]chromen-2-yl)carbamoyl)-[1,1'-biphenyl]-2-carboxylic acid binds to PD-1 and blocks ligands from binding to PD-1 as well as promotes or induces an activating signal through PD-1 that activates the T cell.

### 8. 1,8-bis(phenylthio)anthracene-9,10-dione

Fig. 1H shows the structure of 1,8-bis(phenylthio)anthracene-9,10-dione which bind to PD-1 and modulates signal transduction though PD-1 receptor. In one embodiment, 1,8-bis(phenylthio)anthracene-9,10-dione binds to PD-1 and promotes or induces an activating signal though PD-1 to activate the T cell. In another embodiment, 1,8-bis(phenylthio)anthracene-9,10-dione binds to PD-1 and blocks signal transduction though PD-1 by blocking the interaction of ligands of PD-1 with PD-1 receptor. Exemplary ligands of PD-1 that are blocked by 1,8-bis(phenylthio)anthracene-9,10-dione include, but are not limited to PD-L1 and PD-L2. In another embodiment of 1,8-bis(phenylthio)anthracene-9,10-dione binds to PD-1 and blocks ligands from binding to PD-1 as well as promotes or induces an activating signal through PD-1 that activates the T cell.

### 9. 4-chloro-2-(3-(phenylthio)phenyl)quinoline-6-sulfonyl fluoride

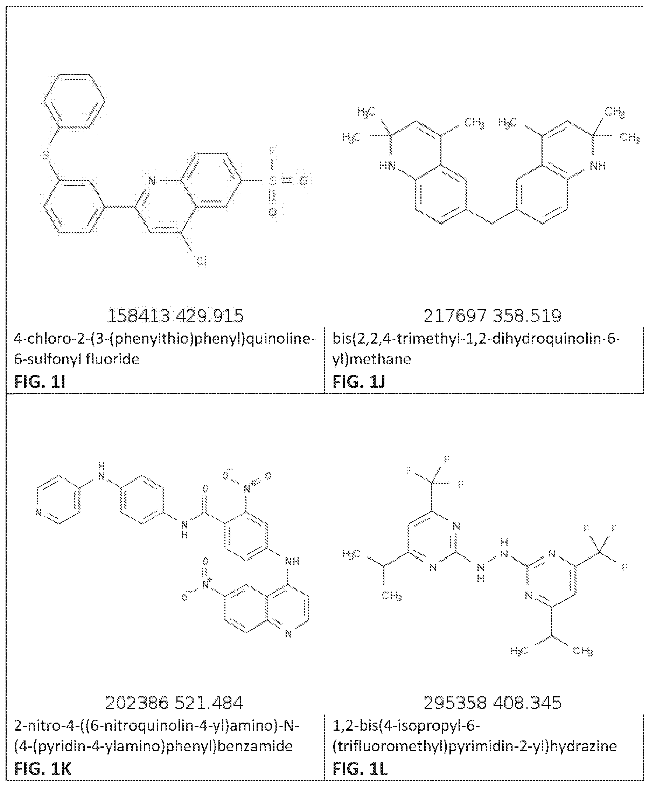

Fig. 1I shows the structure of 4-chloro-2-(3-(phenylthio)phenyl)quinoline-6-sulfonyl fluoride which bind to PD-1 and modulates signal transduction though PD-1 receptor. In one embodiment, 4-chloro-2-(3-(phenylthio)phenyl)quinoline-6-sulfonyl fluoride binds to PD-1 and promotes or induces an activating signal though PD-1 to activate the T cell. In another embodiment, 4-chloro-2-(3-(phenylthio)phenyl)quinoline-6-sulfonyl fluoride binds to PD-1 and blocks signal transduction though PD-1 by blocking the interaction of ligands of PD-1 with PD-1 receptor. Exemplary ligands of PD-1 that are blocked by 4-chloro-2-(3-(phenylthio)phenyl)quinoline-6-sulfonyl fluoride include, but are not limited to PD-L1 and PD-L2. In another embodiment of 4-chloro-2-(3-(phenylthio)phenyl)quinoline-6-sulfonyl fluoride binds to PD-1 and blocks ligands from binding to PD-1 as well as promotes or induces an activating signal through PD-1 that activates the T cell.

### 10. bis(2,2,4-trimethyl-1,2-dihydroquinolin-6-yl)methane

Fig. 1J shows the structure of bis(2,2,4-trimethyl-1,2-dihydroquinolin-6-yl)methane which bind to PD-1 and modulates signal transduction though PD-1 receptor. In one embodiment, bis(2,2,4-trimethyl-1,2-dihydroquinolin-6-yl)methane binds to PD-1 and promotes or induces an activating signal though PD-1 to activate the T cell. In another embodiment, bis(2,2,4-trimethyl-1,2-dihydroquinolin-6-yl)methane binds to PD-1 and blocks signal transduction though PD-1 by blocking the interaction of ligands of PD-1 with PD-1 receptor. Exemplary ligands of PD-1 that are blocked by bis(2,2,4-trimethyl-1,2-dihydroquinolin-6-yl)methane include, but are not limited to PD-L1 and PD-L2. In another embodiment of bis(2,2,4-trimethyl-1,2-dihydroquinolin-6-yl)methane binds to PD-1 and blocks ligands from binding to PD-1 as well as promotes or induces an activating signal through PD-1 that activates the T cell.

### 11. 2-nitro-4-((6-nitroquinolin-4-yl)amino)-N-(4-(pyridin-4-ylamino)phenyl)benzamide

Fig. 1K shows the structure of 2-nitro-4-((6-nitroquinolin-4-yl)amino)-N-(4-(pyridin-4-ylamino)phenyl)benzamide which bind to PD-1 and modulates signal transduction though PD-1 receptor. In one embodiment, 2-nitro-4-((6-nitroquinolin-4-yl)amino)-N-(4-(pyridin-4-ylamino)phenyl)benzamide binds to PD-1 and promotes or induces an activating signal though PD-1 to activate the T cell. In another embodiment, 2-nitro-4-((6-nitroquinolin-4-yl)amino)-N-(4-(pyridin-4-ylamino)phenyl)benzamide binds to PD-1 and blocks signal transduction though PD-1 by blocking the interaction of ligands of PD-1 with PD-1 receptor. Exemplary ligands of PD-1 that are blocked by 2-nitro-4-((6-nitroquinolin-4-yl)amino)-N-(4-(pyridin-4-ylamino)phenyl)benzamide include, but are not limited to PD-L1 and PD-L2. In another embodiment of 2-nitro-4-((6-nitroquinolin-4-yl)amino)-N-(4-(pyridin-4-ylamino)phenyl)benzamide binds to PD-1 and blocks ligands from binding to PD-1 as well as promotes or induces an activating signal through PD-1 that activates the T cell.

### 12. 1,2-bis(4-isopropyl-6-(trifluoromethyl)pyrimidin-2-yl)hydrazine

Fig. 1L shows the structure of 1,2-bis(4-isopropyl-6-(trifluoromethyl)pyrimidin-2-yl)hydrazine which bind to PD-1 and modulates signal transduction though PD-1 receptor. In one embodiment, 1,2-bis(4-isopropyl-6-(trifluoromethyl)pyrimidin-2-yl)hydrazine binds to PD-1 and promotes or induces an activating signal though PD-1 to activate the T cell. In another embodiment, 1,2-bis(4-isopropyl-6-(trifluoromethyl)pyrimidin-2-yl)hydrazine binds to PD-1 and blocks signal transduction though PD-1 by blocking the interaction of ligands of PD-1 with PD-1 receptor. Exemplary ligands of PD-1 that are blocked by 1,2-bis(4-isopropyl-6-(trifluoromethyl)pyrimidin-2-yl)hydrazine include, but are not limited to PD-L1 and PD-L2. In another embodiment of 1,2-bis(4-isopropyl-6-(trifluoromethyl)pyrimidin-2-yl)hydrazine binds to PD-1 and blocks ligands from binding to PD-1 as well as promotes or induces an activating signal through PD-1 that activates the T cell.

### 13. 3-(benzylthio)phenanthro[9,10-e][1,2,4]triazine

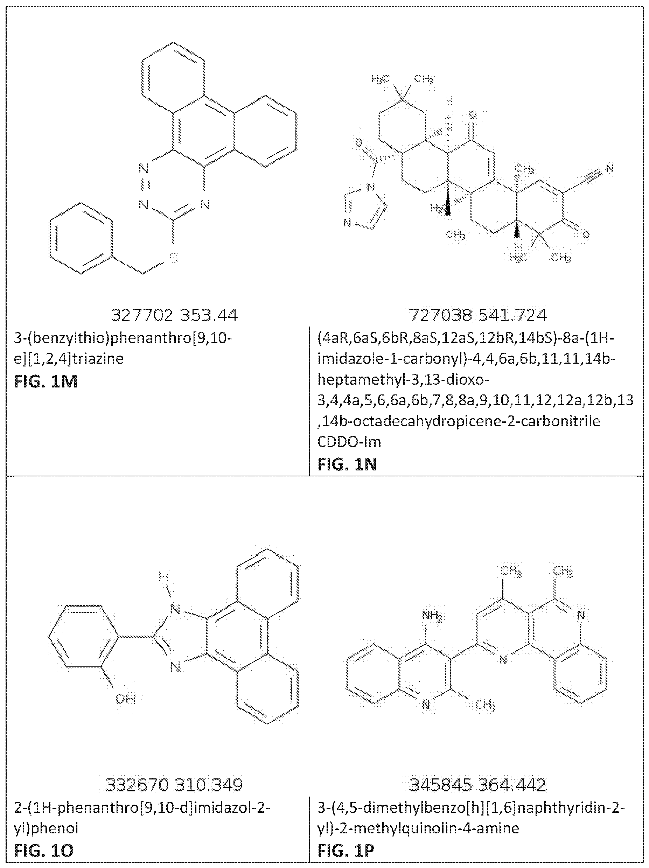

Fig. 1M shows the structure of 3-(benzylthio)phenanthro[9,10-e][1,2,4]triazine which bind to PD-1 and modulates signal transduction though PD-1 receptor. In one embodiment, 3-(benzylthio)phenanthro[9,10-e][1,2,4]triazine binds to PD-1 and promotes or induces an activating signal though PD-1 to activate the T cell. In another embodiment, 3-(benzylthio)phenanthro[9,10-e][1,2,4]triazine binds to PD-1 and blocks signal transduction though PD-1 by blocking the interaction of ligands of PD-1 with PD-1 receptor. Exemplary ligands of PD-1 that are blocked by 3-(benzylthio)phenanthro[9,10-e][1,2,4]triazine include, but are not limited to PD-L1 and PD-L2. In another embodiment of by 3-(benzylthio)phenanthro[9,10-e][1,2,4]triazine binds to PD-1 and blocks ligands from binding to PD-1 as well as promotes or induces an activating signal through PD-1 that activates the T cell.

### 14. (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9,10,11,12,12a,12b,13,14b-octadecahydropicene-2-carbonitrile (CDDO-Im)

Fig. 1N shows the structure of (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9, 10, 11, 12, 12a, 12b, 13, 14b-octadecahydropicene-2-carbonitrile (CDDO-Im) which bind to PD-1 and modulates signal transduction though PD-1 receptor. In one embodiment, (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9, 10, 11, 12, 12a, 12b, 13, 14b-octadecahydropicene-2-carbonitrile (CDDO-Im) binds to PD-1 and promotes or induces an activating signal though PD-1 to activate the T cell. In another embodiment, (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9, 10, 11, 12, 12a, 12b, 13, 14b-octadecahydropicene-2-carbonitrile (CDDO-Im) binds to PD-1 and blocks signal transduction though PD-1 by blocking the interaction of ligands of PD-1 with PD-1 receptor. Exemplary ligands of PD-1 that are blocked by (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9, 10, 11, 12, 12a, 12b, 13, 14b-octadecahydropicene-2-carbonitrile (CDDO-Im) include, but are not limited to PD-L1 and PD-L2. In another embodiment of by (4aR,6aS,6bR,8aS,12aS,12bR,14bS)-8a-(1H-imidazole-1-carbonyl)-4,4,6a,6b,11,11,14b-heptamethyl-3,13-dioxo-3,4,4a,5,6,6a,6b,7,8,8a,9, 10, 11, 12, 12a, 12b, 13, 14b-octadecahydropicene-2-carbonitrile (CDDO-Im) binds to PD-1 and blocks ligands from binding to PD-1 as well as promotes or induces an activating signal through PD-1 that activates the T cell.

### 15. 2-(1H-phenanthro[9,10-d]imidazol-2-yl)phenol

Fig. 1O shows the structure of 2-(1H-phenanthro[9,10-d]imidazol-2-yl)phenol which bind to PD-1 and modulates signal transduction though PD-1 receptor. In one embodiment, 2-(1H-phenanthro[9,10-d]imidazol-2-yl)phenol binds to PD-1 and promotes or induces an activating signal though PD-1 to activate the T cell. In another embodiment, 2-(1H-phenanthro[9,10-d]imidazol-2-yl)phenol binds to PD-1 and blocks signal transduction though PD-1 by blocking the interaction of ligands of PD-1 with PD-1 receptor. Exemplary ligands of PD-1 that are blocked by 2-(1H-phenanthro[9,10-d]imidazol-2-yl)phenol include, but are not limited to PD-L1 and PD-L2. In another embodiment of by 2-(1H-phenanthro[9,10-d]imidazol-2-yl)phenol binds to PD-1 and blocks ligands from binding to PD-1 as well as promotes or induces an activating signal through PD-1 that activates the T cell.

### 16. 3-(4,5-dimethylbenzo[h][1,6]naphthyridin-2-yl)-2-methylquinolin-4-amine

Fig. 1P shows the structure of 2-(1H-phenanthro[9,10-d]imidazol-2-yl)phenol which bind to PD-1 and modulates signal transduction though PD-1 receptor. In one embodiment, 2-(1H-phenanthro[9,10-d]imidazol-2-yl)phenol binds to PD-1 and promotes or induces an activating signal though PD-1 to activate the T cell. In another embodiment, 2-(1H-phenanthro[9,10-d]imidazol-2-yl)phenol binds to PD-1 and blocks signal transduction though PD-1 by blocking the interaction of ligands of PD-1 with PD-1 receptor. Exemplary ligands of PD-1 that are blocked by 2-(1H-phenanthro[9,10-d]imidazol-2-yl)phenol include, but are not limited to PD-L1 and PD-L2. In another embodiment of by 2-(1H-phenanthro[9,10-d]imidazol-2-yl)phenol binds to PD-1 and blocks ligands from binding to PD-1 as well as promotes or induces an activating signal through PD-1 that activates the T cell.

### 17. N-(4-bromonaphthalen-1-yl)-1-hydroxy-2-naphthamide

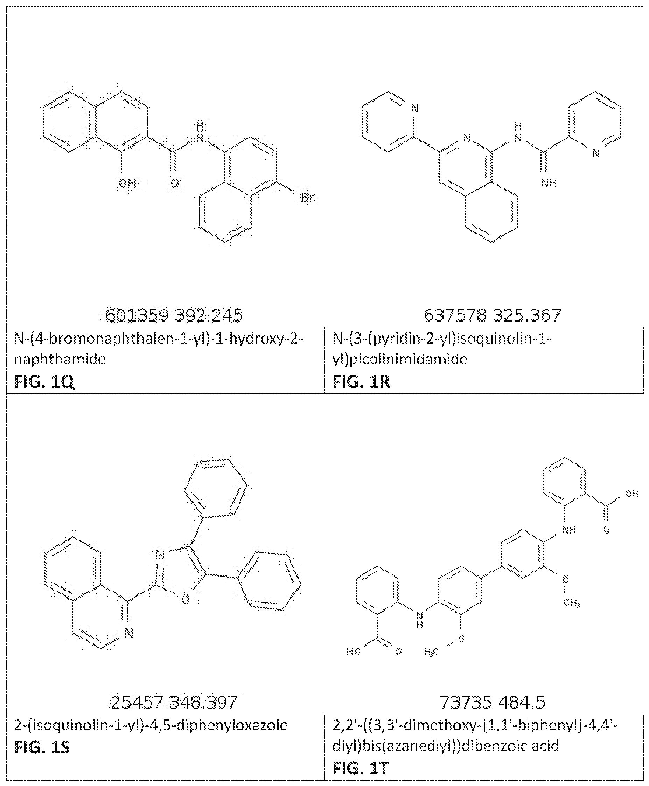

Fig. 1Q shows the structure of N-(4-bromonaphthalen-1-yl)-1-hydroxy-2-naphthamide which bind to PD-1 and modulates signal transduction though PD-1 receptor. In one embodiment, N-(4-bromonaphthalen-1-yl)-1-hydroxy-2-naphthamide binds to PD-1 and promotes or induces an activating signal though PD-1 to activate the T cell. In another embodiment, N-(4-bromonaphthalen-1-yl)-1-hydroxy-2-naphthamide binds to PD-1 and blocks signal transduction though PD-1 by blocking the interaction of ligands of PD-1 with PD-1 receptor. Exemplary ligands of PD-1 that are blocked by N-(4-bromonaphthalen-1-yl)-1-hydroxy-2-naphthamide include, but are not limited to PD-L1 and PD-L2. In another embodiment of N-(4-bromonaphthalen-1-yl)-1-hydroxy-2-naphthamide binds to PD-1 and blocks ligands from binding to PD-1 as well as promotes or induces an activating signal through PD-1 that activates the T cell.

### 18. N-(3-(pyridin-2-yl)isoquinolin-1-yl)picolinimidamide

Fig. 1R shows the structure of N-(3-(pyridin-2-yl)isoquinolin-1-yl)picolinimidamide which bind to PD-1 and modulates signal transduction though PD-1 receptor. In one embodiment, N-(3-(pyridin-2-yl)isoquinolin-1-yl)picolinimidamide binds to PD-1 and promotes or induces an activating signal though PD-1 to activate the T cell. In another embodiment, N-(3-(pyridin-2-yl)isoquinolin-1-yl)picolinimidamide binds to PD-1 and blocks signal transduction though PD-1 by blocking the interaction of ligands of PD-1 with PD-1 receptor. Exemplary ligands of PD-1 that are blocked by N-(3-(pyridin-2-yl)isoquinolin-1-yl)picolinimidamide include, but are not limited to PD-L1 and PD-L2. In another embodiment N-(3-(pyridin-2-yl)isoquinolin-1-yl)picolinimidamide binds to PD-1 and blocks ligands from binding to PD-1 as well as promotes or induces an activating signal through PD-1 that activates the T cell.

### 19. 2-(isoquinolin-1-yl)-4,5-diphenyloxazole

Fig. 1S shows the structure of 2-(isoquinolin-1-yl)-4,5-diphenyloxazole which bind to PD-1 and modulates signal transduction though PD-1 receptor. In one embodiment, 2-(isoquinolin-1-yl)-4,5-diphenyloxazole binds to PD-1 and promotes or induces an activating signal though PD-1 to activate the T cell. In another embodiment, 2-(isoquinolin-1-yl)-4,5-diphenyloxazole binds to PD-1 and blocks signal transduction though PD-1 by blocking the interaction of ligands of PD-1 with PD-1 receptor. Exemplary ligands of PD-1 that are blocked by 2-(isoquinolin-1-yl)-4,5-diphenyloxazole include, but are not limited to PD-L1 and PD-L2. In another embodiment 2-(isoquinolin-1-yl)-4,5-diphenyloxazole binds to PD-1 and blocks ligands from binding to PD-1 as well as promotes or induces an activating signal through PD-1 that activates the T cell.

### 20. 2,2'-((3,3'-dimethoxy-[1,1'-biphenyl]-4,4'-diyl)bis(azanediyl))dibenzoic acid

Fig. 1T shows the structure of 2,2'-((3,3'-dimethoxy-[1,1'-biphenyl]-4,4'-diyl)bis(azanediyl))dibenzoic acid which bind to PD-1 and modulates signal transduction though PD-1 receptor. In one embodiment, 2,2'-((3,3'-dimethoxy-[1,1'-biphenyl]-4,4'-diyl)bis(azanediyl))dibenzoic acid binds to PD-1 and promotes or induces an activating signal though PD-1 to activate the T cell. In another embodiment, 2,2'-((3,3'-dimethoxy-[1,1'-biphenyl]-4,4'-diyl)bis(azanediyl))dibenzoic acid binds to PD-1 and blocks signal transduction though PD-1 by blocking the interaction of ligands of PD-1 with PD-1 receptor. Exemplary ligands of PD-1 that are blocked by 2,2'-((3,3'-dimethoxy-[1,1'-biphenyl]-4,4'-diyl)bis(azanediyl))dibenzoic acid include, but are not limited to PD-L1 and PD-L2. In another embodiment 2,2'-((3,3'-dimethoxy-[1,1'-biphenyl]-4,4'-diyl)bis(azanediyl))dibenzoic acid binds to PD-1 and blocks ligands from binding to PD-1 as well as promotes or induces an activating signal through PD-1 that activates the T cell.

### III. Pharmaceutical Compositions for use in Methods of Treatment

### A. Modulating PD-1 Signal Transduction

The PD-1 modulating compounds and pharmaceutical compositions thereof are generally useful *in vivo* and *ex vivo* as immune response-stimulating therapeutics. In general, the disclosed compositions are useful for treating a subject having or being predisposed to any disease or disorder to which the subject's immune system mounts an immune response.

### 1. Promoting a T cell Activating Signal via PD-1

In one embodiment, the PD-1 modulating compositions modulate PD-1 signal transaction by binding to PD-1 and promoting a T cell activating signal through PD-1. The T cell activating signal causes or promotes T cell activation including one or more of the following: an increase in antigen-specific proliferation of T cells, increased or enhanced cytokine production by T cells, stimulation of differentiation and effector functions of T cells and/or promoting T cell survival), overcoming T cell exhaustion and/or anergy or any combination thereof. In some embodiments, the PD-1 modulating compounds and compositions bind PD-1 and cause or promote signal transduction through PD-1 that activates the T cell and block other ligands from binding to PD-1.

The disclosed PD-1 modulating compositions are useful for stimulating or enhancing an immune response in host for treating cancer or infection by administering to subject an amount of one or more of the PD-1 modulating compositions effective to activate T cells in the subject. The types of cancer that may be treated with the provided compositions and methods include, but are not limited to, the following: bladder, brain, breast, cervical, colo-rectal, esophageal, kidney, liver, lung, nasopharangeal, pancreatic, prostate, skin, stomach, uterine, ovarian, testicular, hematologic, a melanoma, a renal cancer, a myeloma, a thyroid cancer, a lymphoma, a leukemia, or a metastatic lesion of the cancer.

Malignant tumors which may be treated are classified herein according to the embryonic origin of the tissue from which the tumor is derived. Carcinomas are tumors arising from endodermal or ectodermal tissues such as skin or the epithelial lining of internal organs and glands. Sarcomas, which arise less frequently, are derived from mesodermal connective tissues such as bone, fat, and cartilage. The leukemias and lymphomas are malignant tumors of hematopoietic cells of the bone marrow. Leukemias proliferate as single cells, whereas lymphomas tend to grow as tumor masses. Malignant tumors may show up at numerous organs or tissues of the body to establish a cancer.

### 2. Pharmaceutical Compositions for use in Vaccines

The disclosed PD-1 modulating compositions may be administered alone or in combination with any other suitable treatment. In one embodiment one or more of the PD-1 modulating compositions can be administered in conjunction with, or as a component of, a vaccine composition. The disclosed PD-1 modulating compositions can be administered prior to, concurrent with, or after the administration of a vaccine. In one embodiment the PD-1 modulating compositions is administered at the same time as administration of a vaccine.

The disclosed PD-1 modulating compositions may be administered in conjunction with prophylactic vaccines, or therapeutic vaccines, which can be used to initiate or enhance a subject's immune response to a pre-existing antigen, such as a tumor antigen in a subject with cancer.

The desired outcome of a prophylactic, therapeutic or de-sensitized immune response may vary according to the disease, according to principles well known in the art. Similarly, immune responses against cancer, allergens or infectious agents may completely treat a disease, may alleviate symptoms, or may be one facet in an overall therapeutic intervention against a disease. For example, the stimulation of an immune response against a cancer may be coupled with surgical, chemotherapeutic, radiologic, hormonal and other immunologic approaches in order to affect treatment.

### 3. Adjuvant Therapy

The disclosed PD-1 modulating compositions may be used to overcome tolerance to antigens, and thereby treat cancer. Appropriate targeting of co-signaling pathways can lead to activation of T cells and overcome tolerance to tumor antigens. The PD-1 modulating composition may be administered in an effective amount to overcome antigen tolerance. The PD-1 modulating compositions can also inhibit or reduce PD-1 negative signaling to promote, enhance, or amplify T cell responses and overall immunity following administration of a first therapeutic agent or a response to a poorly immunogenic antigen such as a tumor associated antigen. PD-1 modulating compositions may also be administered passively following primary treatment, vaccination, or killing of the tumor (antibody-mediated, with chemotherapy or radiation or any combination thereof). The PD-1 modulating compositions are believed to enhance/boost the primary response resulting in a robust and long-lasting protective response to the tumor.

Treatment that is administered in addition to a first therapeutic agent to eradicate tumors is referred to as adjuvant therapy. Adjuvant treatment is given to augment the primary treatment, such as surgery or radiation, to decrease the chance that the cancer will recur. This additional treatment can result in an amplification of the primary response as evidenced by a more potent and/or prolonged response.

There are five main types of adjuvant therapy (note that some of these are also used as primary/monotherapy as well): 1.) Chemotherapy that uses drugs to kill cancer cells, either by preventing them from multiplying or by causing the cells to self-destruct., 2.) Hormone therapy
to reduce hormone production and prevent the cancer from growing, 3.) Radiation therapy that uses high-powered rays to kill cancer cells, 4.) Immunotherapy that attempts to influence the body's own immune system to attack and eradicate any remaining cancer cells. Immunotherapy can either stimulate the body's own defenses (cancer vaccines) or supplement them (passive administration of antibodies or immune cells), or 5.) Targeted therapy that targets specific molecules present within cancer cells, leaving out normal, healthy cells. For example, many cases of breast cancer are caused by tumors that produce too much of a protein called HER2. Trastuzumab (HERCEPTIN^{®}) is used as adjuvant therapy that targets HER2 positive tumors.

Typically adjuvant treatments are co-administered or given in conjunction with primary treatments to induce multiple mechanisms and increase the chances of eradicating the tumor. Immunotherapy, and vaccines in particular, offer the unique advantages of inducing a sustained antitumor effect with exquisite specificity and with the ability to circumvent existing immune tolerance. It has been discovered that delaying "adjuvant therapy" maximizes the response and increases the chances of eradicating tumors.

In one embodiment, PD-1 modulating compositions as described herein, are administered following administration of a first therapeutic agent such as a cancer therapeutic agent. The timing of the administration of the adjuvant can range from day 0 to day 14 after the primary treatment and can include single or multiple treatments. In certain embodiments, the PD-1 modulating composition is administered 1, 2, 3, 4, 5, 6, 7, 8, 9, 10, 11, 12, 13, or 14 days after administration of the primary treatment. The adjuvant is preferably administered systemically to the patient (IV, IM or SQ).

The choice of PD-1 modulating composition for use to enhance the immune response may depend on the original mode of primary treatment. Therefore specific combinations of therapeutics and PD-1 modulating compositions may be required for optimum efficacy. The PD-1 modulating compositions may be optimized for the type of cancer, for example solid versus liquid tumor for example using affinity maturation.

Administration is not limited to the treatment of an existing tumor or infectious disease but can also be used to prevent or lower the risk of developing such diseases in an individual, i.e., for prophylactic use. Potential candidates for prophylactic vaccination include individuals with a high risk of developing cancer, i.e., with a personal or familial history of certain types of cancer.

Another embodiment provides a pharmaceutical composition for use in a method for increasing the population of tumor infiltrating leukocytes in a subject by administering to the subject an effective amount of PD-1 modulating compositions to enhance activation of the subject's T cells.

### B. Combination Therapies

The disclosed PD-1 modulating compositions can be administered to a subject in need thereof alone or in combination with one or more additional therapeutic agents or combinations of the recited PD-1 modulating compositions. The additional therapeutic agents are selected based on the condition, disorder or disease to be treated. For example, PD-1 modulating compositions can be co-administered with one or more additional agents that function to enhance or promote an immune response.

### 1. Chemotherapeutic Agents

The PD-1 modulating compositions can also be combined with one or more additional therapeutic agents. Representative therapeutic agents include, but are not limited to chemotherapeutic agents and pro-apoptotic agents. Representative chemotherapeutic agents include, but are not limited to amsacrine, bleomycin, busulfan, capecitabine, carboplatin,carmustine, chlorambucil, cisplatin, cladribine, clofarabine, crisantaspase, cyclophosphamide, cytarabine, dacarbazine, dactinomycin, daunorubicin, docetaxel, doxorubicin, epirubicin, etoposide, fludarabine, fluorouracil, gemcitabine, hydroxycarbamide, idarubicin, ifosfamide, irinotecan, leucovorin, liposomal doxorubicin, liposomal daunorubicin , lomustine, melphalan, mercaptopurine, mesna, methotrexate, mitomycin, mitoxantrone, oxaliplatin, paclitaxel, pemetrexed, pentostatin, procarbazine, raltitrexed, satraplatin, streptozocin, tegafur-uracil, temozolomide, teniposide, thiotepa, tioguanine, topotecan, treosulfan, vinblastine, vincristine, vindesine, vinorelbine, or a combination thereof. Representative pro-apoptotic agents include, but are not limited to fludarabinetaurosporine, cycloheximide, actinomycin D, lactosylceramide, 15d-PGJ(2) and combinations thereof.

In certain embodiments, more than one multivalent immunomodulatory composition can be used in combination to increase or enhance an immune response in a subject.

### 2. Potentiating Agents

The disclosed PD-1 modulating compounds and compositions can be administered in combination with or alternation with agents that promote or enhance an immune response in a subject. These additional agents are collective referred to as immunopotentiating agents. Representative immunopotentiating agents that can be used with the disclosed PD-1 modulating compounds include, but are not limited to bacillus Camette-Guérin (BCG), killed *Corynebacterium parvum*, mycobacterial cell walls, CpG oligodeoxynucleotides, pegfilgrastim, filgrastim, tbo-filgrastim, sargramostim, aldesleukin, oprelvekin, interferon beta-1a, interferon alfacon-1, interferon gamma-1b, interferon alfa-n3, interferon beta-1b, peginterferon beta-1a, glatiramer, pegademase bovine, plerixafor, cyclophosphamide, ifosfamide, perfosfamide, trophosphamide, and combinations thereof.

### C. Adoptive Transfer

Adoptive T-cell therapy is a promising strategy for the treatment of patients with established tumors but is often limited to specific cancers where tumor-infiltrating lymphocytes, the source of T cells for *ex vivo* culture, can be obtained. One embodiment provides a pharmaceutical composition for use in a method for treating cancer by administering an effective amount of the composition to inhibit or reduce PD-1 receptor mediated inhibitory signal transduction in a T cell in combination with adoptive T-cell therapy of antigen specific T cells. The adoptive T-cell transfer can be administered to the subject prior to or following administration of the PD-1 modulating composition or added to the cells *ex vivo.*

Antigen-specific T-cell lines can be generated by *in vitro* stimulation with antigen followed by nonspecific expansion on CD3/CD28 beads. The ability to expand antigen-specific T cells can be assessed using IFN-gamma and granzyme B enzyme-linked immunosorbent spot. The phenotype of the resultant T-cell lines can be evaluated by flow cytometry, including the presence of FOXP3-expressing CD4(+) T cells. Amplification of antigen-specific T cell populations from Peripheral Blood Mononuclear Cells (PBMCs) is usually performed through repeated in-vitro stimulation with optimal length antigenic peptides in the presence of IL-2. Low doses of IL-2 (between 10 and 50 U/ml) have been used traditionally to avoid the activation/expansion of lymphokine-activated killer cells, as revealed in chromium release assays that were commonly employed to monitor specific T cell expansion. Concentrations of antigenic peptides can be 0.1-10 µM.

### 1. Tumor-specific and Tumor-associated Antigens

Antigens useful for expanding T cells can be obtained from biopsies of tumors from the subject to be treated. The antigens can be biochemically purified from the tumor biopsy. Alternatively, the antigens can be recombinant polypeptides. The antigen expressed by the tumor may be specific to the tumor, or may be expressed at a higher level on the tumor cells as compared to non-tumor cells. Antigenic markers such as serologically defined markers known as tumor associated antigens, which are either uniquely expressed by cancer cells or are present at markedly higher levels (e.g., elevated in a statistically significant manner) in subjects having a malignant condition relative to appropriate controls, are contemplated for use in certain embodiments.

Tumor-associated antigens may include, for example, cellular oncogene-encoded products or aberrantly expressed proto-oncogene-encoded products (e.g., products encoded by the neu, ras, trk, and kit genes), or mutated forms of growth factor receptor or receptor-like cell surface molecules (e.g., surface receptor encoded by the c-erb B gene). Other tumor-associated antigens include molecules that may be directly involved in transformation events, or molecules that may not be directly involved in oncogenic transformation events but are expressed by tumor cells (e.g., carcinoembryonic antigen, CA-125, melonoma associated antigens, etc.) (see, e.g., U.S. Pat. No. 6,699,475; Jager, et al., Int. J. Cancer, 106:817-20 (2003); Kennedy, et al., Int. Rev. Immunol., 22:141-72 (2003); Scanlan, et al. Cancer Immun., 4:1 (2004)).

Genes that encode cellular tumor associated antigens include cellular oncogenes and proto-oncogenes that are aberrantly expressed. In general, cellular oncogenes encode products that are directly relevant to the transformation of the cell, and because of this, these antigens are particularly preferred targets for immunotherapy. An example is the tumorigenic neu gene that encodes a cell surface molecule involved in oncogenic transformation. Other examples include the ras, kit, and trk genes. The products of proto-oncogenes (the normal genes which are mutated to form oncogenes) may be aberrantly expressed (e.g., overexpressed), and this aberrant expression can be related to cellular transformation. Thus, the product encoded by proto-oncogenes can be targeted. Some oncogenes encode growth factor receptor molecules or growth factor receptor-like molecules that are expressed on the tumor cell surface. An example is the cell surface receptor encoded by the c-erbB gene. Other tumor-associated antigens may or may not be directly involved in malignant transformation. These antigens, however, are expressed by certain tumor cells and may therefore provide effective targets. Some examples are carcinoembryonic antigen (CEA), CA 125 (associated with ovarian carcinoma), and melanoma specific antigens.

In ovarian and other carcinomas, for example, tumor associated antigens are detectable in samples of readily obtained biological fluids such as serum or mucosal secretions. One such marker is CA125, a carcinoma associated antigen that is also shed into the bloodstream, where it is detectable in serum (e.g., Bast, et al., N. Eng. J. Med., 309:883 (1983); Lloyd, et al., Int. J. Canc., 71:842 (1997). CA125 levels in serum and other biological fluids have been measured along with levels of other markers, for example, carcinoembryonic antigen (CEA), squamous cell carcinoma antigen (SCC), tissue polypeptide specific antigen (TPS), sialyl TN mucin (STN), and placental alkaline phosphatase (PLAP), in efforts to provide diagnostic and/or prognostic profiles of ovarian and other carcinomas (e.g., Sarandakou, et al., Acta Oncol., 36:755 (1997); Sarandakou, et al., Eur. J. Gynaecol. Oncol., 19:73 (1998); Meier, et al., Anticancer Res., 17(4B):2945 (1997); Kudoh, et al., Gynecol. Obstet. Invest., 47:52 (1999)). Elevated serum CA125 may also accompany neuroblastoma (e.g., Hirokawa, et al., Surg. Today, 28:349 (1998), while elevated CEA and SCC, among others, may accompany colorectal cancer (Gebauer, et al., Anticancer Res., 17(4B):2939 (1997)).

The tumor associated antigen, mesothelin, defined by reactivity with monoclonal antibody K-1, is present on a majority of squamous cell carcinomas including epithelial ovarian, cervical, and esophageal tumors, and on mesotheliomas (Chang, et al., Cancer Res., 52:181 (1992); Chang, et al., Int. J. Cancer, 50:373 (1992); Chang, et al., Int. J. Cancer, 51:548 (1992); Chang, et al., Proc. Natl. Acad. Sci. USA, 93:136 (1996); Chowdhury, et al., Proc. Natl. Acad. Sci. USA, 95:669 (1998)). Using MAb K-1, mesothelin is detectable only as a cell-associated tumor marker and has not been found in soluble form in serum from ovarian cancer patients, or in medium conditioned by OVCAR-3 cells (Chang, et al., Int. J. Cancer, 50:373 (1992)). Structurally related human mesothelin polypeptides, however, also include tumor-associated antigen polypeptides such as the distinct mesothelin related antigen (MRA) polypeptide, which is detectable as a naturally occurring soluble antigen in biological fluids from patients having malignancies.

A tumor antigen may include a cell surface molecule. Tumor antigens of known structure and having a known or described function (see above).

### 2. Antigens Associated With Tumor Neovasculature

Protein therapeutics can be ineffective in treating tumors because they are inefficient at tumor penetration.

The antigen may be specific to tumor neovasculature or may be expressed at a higher level in tumor neovasculature when compared to normal vasculature. Exemplary antigens that are over-expressed by tumor-associated neovasculature as compared to normal vasculature include, but are not limited to, VEGF/KDR, Tie2, vascular cell adhesion molecule (VCAM), endoglin and α5β3 integrin/vitronectin. Other antigens that are over-expressed by tumor-associated neovasculature as compared to normal vasculature are known to those of skill in the art and are suitable for targeting by the disclosed fusion proteins.

### D. Treatment of Infections

The disclosed PD-1 modulating compounds and compositions can be used to treat infections including but not limited to microbial infections such as bacterial, fungal, viral and mycoplasma infections. The PD-1 modulating composition can also be used to treat parasitic infections. One embodiment provides a pharmaceutical composition for use in a method for treating an infection in a subject by administering to the subject one or more PD-1 modulating compounds or compositions in an amount effective to promote or induce an activating signal through the PD-1 receptor activate T cells in the subject.

### IV. Formulations and Administration

The disclosed PD-1 modulating compounds can be formulated as pharmaceutical compositions for administration by parenteral (intramuscular, intraperitoneal, intravenous (IV) or subcutaneous injection), transdermal (either passively or using iontophoresis or electroporation), or transmucosal (nasal, vaginal, rectal, or sublingual) routes of administration or using bioerodible inserts and can be formulated in dosage forms appropriate for each route of administration.

In some *in vivo* approaches, the compositions disclosed herein are administered to a subject in a therapeutically effective amount. As used herein the term "effective amount" or "therapeutically effective amount" means a dosage sufficient to treat, inhibit, or alleviate one or more symptoms of the disorder being treated or to otherwise provide a desired pharmacologic and/or physiologic effect. The precise dosage will vary according to a variety of factors such as subject-dependent variables (e.g., age, immune system health, etc.), the disease, and the treatment being effected.

As further studies are conducted, information will emerge regarding appropriate dosage levels for treatment of various conditions in various patients, and the ordinary skilled worker, considering the therapeutic context, age, and general health of the recipient, will be able to ascertain proper dosing. The selected dosage depends upon the desired therapeutic effect, on the route of administration, and on the duration of the treatment desired. Generally dosage levels of 0.001 to 10 mg/kg of body weight daily are administered to mammals. Generally, for intravenous injection or infusion, the dosage may be lower.

In certain embodiments, the PD-1 modulating compositions for use herein are administered locally, for example by injection directly into a site to be treated. Typically, the injection causes an increased localized concentration of the PD-1 modulating compositions which is greater than that which can be achieved by systemic administration. The PD-1 modulating compositions can be combined with a matrix to assist in creating an increased localized concentration of the PD-1 modulating compositions by reducing the passive diffusion of the compounds out of the site to be treated.

### A. Formulations for Parenteral Administration

In one embodiment, the PD-1 modulating compositions are administered in an aqueous solution, by parenteral injection. The formulation may also be in the form of a suspension or emulsion. In general, pharmaceutical compositions are provided including effective amounts of a PD-1 modulating compound and optionally include pharmaceutically acceptable diluents, preservatives, solubilizers, emulsifiers, adjuvants and/or carriers. Such compositions include diluents such as sterile water, buffered saline of various buffer content (*e.g*., Tris-HCl, acetate, phosphate), pH and ionic strength; and optionally, additives such as detergents and solubilizing agents (*e.g*., TWEEN^{®} 20, TWEEN^{®} 80 also referred to as polysorbate 20 or 80), anti-oxidants (*e.g.*, ascorbic acid, sodium metabisulfite), and preservatives (*e.g*., Thimersol, benzyl alcohol) and bulking substances (*e.g*., lactose, mannitol). Examples of non-aqueous solvents or vehicles are propylene glycol, dextrin, polyethylene glycol, vegetable oils, such as olive oil and corn oil, gelatin, and injectable organic esters such as ethyl oleate. The formulations may be lyophilized and redissolved/resuspended immediately before use. The formulation may be sterilized by, for example, filtration through a bacteria retaining filter, by incorporating sterilizing agents into the compositions, by irradiating the compositions, or by heating the compositions.

### B. Formulations for Topical Administration

The PD-1 modulating compositions can be applied topically including application to the lungs, nasal, oral (sublingual, buccal), vaginal, or rectal mucosa. The compositions can be delivered to the lungs while inhaling and traverse across the lung epithelial lining to the blood stream when delivered either as an aerosol or spray dried particles having an aerodynamic diameter of less than about 5 microns.

A wide range of mechanical devices designed for pulmonary delivery of therapeutic products can be used, including but not limited to nebulizers, metered dose inhalers, and powder inhalers, all of which are familiar to those skilled in the art. Some specific examples of commercially available devices are the Ultravent nebulizer (Mallinckrodt Inc., St. Louis, Mo.); the Acorn II nebulizer (Marquest Medical Products, Englewood, Colo.); the Ventolin metered dose inhaler (Glaxo Inc., Research Triangle Park, N.C.); and the Spinhaler powder inhaler (Fisons Corp., Bedford, Mass.). Nektar, Alkermes and Mannkind all have inhalable insulin powder preparations approved or in clinical trials where the technology could be applied to the formulations described herein.

Formulations for administration to the mucosa will typically be spray dried PD-1 modulating compound particles, which may be incorporated into a tablet, gel, capsule, suspension or emulsion. Standard pharmaceutical excipients are available from any formulator. Oral formulations may be in the form of chewing gum, gel strips, tablets or lozenges.

Transdermal formulations may also be prepared. These will typically be ointments, lotions, sprays, or patches, all of which can be prepared using standard technology. Transdermal formulations will require the inclusion of penetration enhancers.

### C. Controlled Delivery Polymeric Matrices

The PD-1 modulating compositions may also be administered in controlled release formulations. Controlled release polymeric devices can be made for long term release systemically following implantation of a polymeric device (rod, cylinder, film, disk) or injection (microparticles). The matrix can be in the form of microparticles such as microspheres, where peptides are dispersed within a solid polymeric matrix or microcapsules, where the core is of a different material than the polymeric shell, and the peptide is dispersed or suspended in the core, which may be liquid or solid in nature. Unless specifically defined herein, microparticles, microspheres, and microcapsules are used interchangeably. Alternatively, the polymer may be cast as a thin slab or film, ranging from nanometers to four centimeters, a powder produced by grinding or other standard techniques, or even a gel such as a hydrogel.

Either non-biodegradable or biodegradable matrices can be used for delivery of PD-1 modulating compositions, although biodegradable matrices are preferred. These may be natural or synthetic polymers, although synthetic polymers are preferred due to the better characterization of degradation and release profiles. The polymer is selected based on the period over which release is desired. In some cases linear release may be most useful, although in others a pulse release or "bulk release" may provide more effective results. The polymer may be in the form of a hydrogel (typically in absorbing up to about 90% by weight of water), and can optionally be crosslinked with multivalent ions or polymers.

The matrices can be formed by solvent evaporation, spray drying, solvent extraction and other methods known to those skilled in the art. Bioerodible microspheres can be prepared using any of the methods developed for making microspheres for drug delivery, for example, as described by Mathiowitz and Langer, J. Controlled Release, 5:13-22 (1987); Mathiowitz, et al., Reactive Polymers, 6:275-283 (1987); and Mathiowitz, et al., J. Appl. Polymer Sci., 35:755-774 (1988).

The devices can be formulated for local release to treat the area of implantation or injection--which will typically deliver a dosage that is much less than the dosage for treatment of an entire body--or systemic delivery. These can be implanted or injected subcutaneously, into the muscle, fat, or swallowed.

## Claims

1. A pharmaceutical composition comprising
(a) one or more PD-1 modulating compounds selected from 1-(1H-benzo[d] [1,2,3]triazol-1-yl)anthracene-9,10-dione; 1,1'-(oxybis(4,1-phenylene))bis(3-(2-chlorophenyl)urea); 5,5'diphenyl-2,2',3,3'-tetrahydro-2,2'-bibenzo[d]oxazole; 2-(isoquinolin-1-yl)-5-phenyl-4-(p-tolyl)oxazole; 2'-((6-oxo-5,6-dihydrophenanthridin-3-yl)carbamoyl)-[1,1'-biphenyl]-2-carboxylic acid; 2'-((6-oxo-6H-benzo[c]chromen-2-yl)carbamoyl)-[1,1'-biphenyl]-2-carboxylic acid; 3-(4-chloro-6-phenoxy-1,3,5-triazin-2-yl)-1-phenyl-1H-indole; 1,8-bis(phenylthio)anthracene-9,10-dione; 4-chloro-2-(3-(phenylthio)phenyl)quinoline-6-sulfonyl fluoride; bis(2,2,4-trimethyl-1,2-dihydroquinolin-6-yl)methane; 2-nitro-4-((6-nitroquinolin-4-yl)amino)-N-(4-(pyridin-4-ylamino)phenyl)benzamide; 1,2-bis(4-isopropyl-6-(trifluoromethyl)pyrimidin-2-yl)hydrazine; 3-(benzylthio)phenanthro[9,10-e] [ 1,2,4]triazine; (4aR,6aS,6bR,8aS,12aS,12bR,14bS)-8a-(1H-imidazole-1-carbonyl)-4,4,6a,6b, 11,11,14b-heptamethyl-3, 13-dioxo-3,4,4a,S,6,6a,6b, 7,8,8a,9, 10, 11, 12, 12a, 12b, 13, 14b-octadecahydropicene-2-carbonitrile (CDDO-Im); 2-(1H-phenanthro[9,10-d]imidazol-2-yl)phenol; 3-(4,5-dimethylbenzo[h][1,6]naphthyridin-2-yl)-2-methylquinolin-4-amine; N-(4-bromonaphthalen-1-yl)-1-hydroxy-2-naphthamide; N-(3-(pyridin-2-yl)isoquinolin-1-yl)picolinimidamide; 2-(isoquinoln-1-yl)-4,5-diphenyloxazole; 2,2'-((3,3'-dimethoxy-[1,1'-biphenyl]-4,4'-diyl)bis(azanediyl))dibenzoic acid; or solvate, or pharmaceutically acceptable salt thereof; and
(b) a pharmaceutically acceptable carrier.

2. The pharmaceutical composition of claim 1 for use in a method of treating cancer in a subject, the method comprising administering to the subject an effective amount of the composition to induce or promote T cell activation in the subject.

3. The pharmaceutical composition for use according to claim 2, wherein the cancer is selected from the group consisting of bladder, brain, breast, cervical, colo-rectal, esophageal, kidney, liver, lung, nasopharangeal, pancreatic, prostate, skin, stomach, uterine, ovarian, testicular, hematologic, a melanoma, a renal cancer, a myeloma, a thyroid cancer, a lymphoma, a leukemia, or a metastatic lesion of the cancer.

4. The pharmaceutical composition of claim 1 for use in a method of treating an infection in a subject in need thereof, the method comprising administering to the subject an effective amount of the composition to promote or induce T cell activation in the subj ect.

5. The pharmaceutical composition for use according to claim 4, wherein the infection is a microbial infection, optionally wherein the infection is bacterial, fungal, or viral.

6. The pharmaceutical composition for use according to claim 4, wherein the infection is parasitic.

7. The pharmaceutical composition for use according to claim 2, wherein the composition is administered in conjunction with, or as a component of, a vaccine.

8. The pharmaceutical composition for use according to claim 2, wherein the composition is administered as an adjuvant treatment in conjunction with a primary cancer treatment.

9. The pharmaceutical composition for use according to claim 2, wherein the composition is administered as an adjuvant treatment following administration of a first therapeutic agent,
optionally wherein the composition is administered to the subject 1, 2, 3, 4, 5, 6, 7, 8, 9, 10, 11, 12, 13, or 14 days after administration of the first therapeutic agent.

10. The pharmaceutical composition for use according to any one of claims 2-9, wherein the composition is administered to the subject systemically.

11. The pharmaceutical composition for use according to any one of claims 2-9, wherein the composition is administered to the subject locally at a site to be treated.

## Patentansprüche

1. Pharmazeutische Zusammensetzung, umfassend
(a) eine oder mehrere PD-1 modulierende Verbindungen, ausgewählt aus 1-(1H-Benzo[d] [1,2,3]triazol-1-yl)anthracen-9,10-dion, 1,1'-(Oxybis(4,1-phenylen))bis(3-(2-chlorphenyl)urea); 5,5'-Diphenyl-2,2',3,3'-tetrahydro-2,2'-bibenzo[d]oxazol; 2-(Isochinolin-1-yl)-5-phenyl-4-(p-tolyl)oxazol, 2'-((6-oxo-5,6-Dihydrophenanthridin-3-yl)carbamoyl)-[1,1'-biphenyl]-2-carboxylsäure, 2'-((6-oxo-6H-Benzo[c]chromen-2-yl)carbamoyl)-[1,1'-biphenyl]-2-carboxylsäure, 3-(4-Chlor-6-phenoxy-1,3,5-triazin-2-yl)-1-phenyl-1H-indol; 1,8-bis(Phenylthio)anthracen-9,10-dion; 4-Chlor-2-(3-(phenylthio)phenyl)chinolin-6-sulfonylfluorid; bis(2,2,4-Trimethyl-1,2-dihydrochinolin-6-yl)methan; 2-Nitro-4-((6-nitrochinolin-4-yl)amino)-N-(4-(pyridin-4-ylamino)phenyl)benzamid; 1,2-bis(4-Isopropyl-6-(trifluormethyl)pyrimidin-2-yl)hydrazin; 3-(Benzylthio)phenanthro[9,10-e][1,2,4]triazin; (4aR,6aS,6bR,8aS,12aS,12bR,14bS)-8a-(1H-Imidazol-1-carbonyl)-4,4,6a,6b,11,11,14bheptamethyl-3,13-dioxo-3,4,4a,5,6,6a,6b,7,8,8a,9,10,11,12,12a,12b,13,14boctadecahydropicen-2-carbonitril (CDDO-1m); 2-(1H-Phenanthro[9,10-d]imidazol-2-yl)phenol; 3-(4,5-dimethylbenzo[h][1,6]naphthyridin-2-yl)-2-methylchinolin-4-amin, N(4-Bromonaphthalen-1-yl)-1-hydroxy-2-naphthamid, N-(3-(Pyridin-2-yl)isochinolin-1-yl)picolinimidamid; 2-(isochinolin-1-yl)-4,5-diphenyloxazol, 2,2'-((3,3'-Dimethoxy-[1,1'-biphenyl]-4,4'-diyl)bis(azanediyl))dibenzoesäure; oder Solvat, oder ein pharmazeutisch annehmbares Salz davon; und
(b) einen pharmazeutisch annehmbaren Träger.

2. Pharmazeutische Zusammensetzung nach Anspruch 1 zur Verwendung in einem Verfahren zur Behandlung von Krebs in einem Subjekt, wobei das Verfahren die Verabreichung einer wirksamen Menge der Zusammensetzung an das Subjekt umfasst, um die T-Zellaktivierung in dem Subjekt zu induzieren oder zu fördern.

3. Pharmazeutische Zusammensetzung zur Verwendung nach Anspruch 2, wobei der Krebs ausgewählt ist aus der Gruppe bestehend aus Blasen-, Gehirn-, Brust-, Gebärmutterhals-, Kolorektal-, Speiseröhren-, Nieren-, Leber-, Lungen-, Nasopharynx- , Bauchspeicheldrüsen-, Prostata-, Haut-, Magen-, Gebärmutter-, Eierstock-, Hoden-, hämatologischem Krebs, einem Melanom, Nierenkrebs, einem Myelom, Schilddrüsenkrebs, einem Lymphom, einer Leukämie oder einer metastatischen Läsion des Krebses.

4. Pharmazeutische Zusammensetzung nach Anspruch 1 zur Verwendung in einem Verfahren zur Behandlung einer Infektion in einem Subjekt, das dessen bedarf, wobei das Verfahren die Verabreichung einer wirksamen Menge der Zusammensetzung an das Subjekt umfasst, um die T-Zell-Aktivierung in dem Subjekt zu fördern oder zu induzieren.

5. Pharmazeutische Zusammensetzung zur Verwendung nach Anspruch 4, wobei die Infektion eine mikrobielle Infektion ist, optional wobei die Infektion bakteriell, pilzartig oder viral ist.

6. Pharmazeutische Zusammensetzung zur Verwendung nach Anspruch 4, wobei die Infektion parasitär ist.

7. Pharmazeutische Zusammensetzung zur Verwendung nach Anspruch 2, wobei die Zusammensetzung in Verbindung mit oder als Komponente eines Impfstoffs verabreicht wird.

8. Pharmazeutische Zusammensetzung zur Verwendung nach Anspruch 2, wobei die Zusammensetzung als adjuvante Behandlung in Verbindung mit einer primären Krebsbehandlung verabreicht wird.

9. Pharmazeutische Zusammensetzung zur Verwendung nach Anspruch 2, wobei die Zusammensetzung als adjuvante Behandlung im Anschluss an die Verabreichung eines ersten therapeutischen Mittels verabreicht wird, wobei die Zusammensetzung dem Subjekt optional 1, 2, 3, 4, 5, 6, 7, 8, 9, 10, 11, 12, 13 oder 14 Tage nach Verabreichung des ersten therapeutischen Mittels verabreicht wird.

10. Pharmazeutische Zusammensetzung zur Verwendung nach einem der Ansprüche 2 - 9, wobei die Zusammensetzung dem Subjekt systemisch verabreicht wird.

11. Pharmazeutische Zusammensetzung zur Verwendung nach einem der Ansprüche 2 - 9, wobei die Zusammensetzung dem Subjekt lokal an einer zu behandelnden Stelle verabreicht wird.

## Revendications

1. Composition pharmaceutique, comprenant
(a) un ou plusieurs composés à modulation de PD-1 sélectionnés parmi : 1-(1H-benzo[d][1,2,3]triazol-1-yl)anthracène-9,10-dione ; 1,1'-(oxybis(4,1-phénylène))bis(3-(2-chlorophényl)urée) ; 5,5'diphényl-2,2',3,3'-tétrahydro-2,2'-bibenzo[d]oxazole ; 2-(isoquinolin-1-yl)-5-phényl-4-(p-tolyl)oxazole ; acide 2'-((6-oxo-5,6-dihydrophénanthridin-3-yl)carbamoyl)-[1,1'-biphényl]-2-carboxylique ; acide 2'-((6-oxo-6H-benzo[c]chromén-2-yl)carbamoyl)-[1,1'-biphényl]-2-carboxylique ; 3-(4-chloro-6-phénoxy-1,3,5-triazin-2-yl)-1-phényl-1H-indole ; 1,8-bis(phénylthio)anthracène-9,10-dione ; fluorure de 4-chloro-2-(3-(phénylthio)phényl)quinoline-6-sulfonyle ; bis(2,2,4-triméthyl-1,2-dihydroquinolin-6-yl)méthane ; 2-nitro-4-((6-nitroquinolin-4-yl)amino)-N-(4-(pyridin-4-ylamino)phényl)benzamide ; 1,2-bis(4-isopropyl-6-(trifluorométhyl)pyrimidin-2-yl)hydrazine ; 3-(benzylthio)phénanthro[9,10-e][1,2,4]triazine; (4aR,6aS,6bR,8aS,12aS,12bR,14bS)-8a-(1H-imidazole-1-carbonyl)-4,4,6a,6b,11,11,14b-heptaméthyl-3,13-dioxo-3,4,4a, 5, 6,6a, 6b, 7, 8, 8a, 9,10,11,12,12a,12b,13,14b-octadécahydropicène-2-carbonitrile (CDDO-Im) ; 2-(1H-phénanthro[9,10-d]imidazol-2-yl)phénol ; 3-(4,5-diméthylbenzo[h][1,6]naphtyridin-2-yl)-2-méthylquinolin-4-amine ; N(4-bromonaphtalèn-1-yl)-1-hydroxy-2-naphtamide ; N-(3-(pyridin-2-yl)isoquinolin-1-yl)picolinimidamide ; 2-(isoquinoln-1-yl)-4,5-diphényloxazole ; acide 2,2'-((3,3'-diméthoxy-[1,1'-biphényl]-4,4'-diyl)bis(azanédiyl))dibenzoïque ; ou solvate, ou sel pharmaceutiquement acceptable de ceux-ci ; et
(b) un vecteur pharmaceutiquement acceptable.

2. Composition pharmaceutique selon la revendication 1 pour l'utilisation dans un procédé de traitement de cancer chez un sujet, le procédé comprenant l'administration, au sujet, d'une quantité efficace de la composition pour provoquer ou favoriser une activation des cellules T chez le sujet.

3. Composition pharmaceutique pour l'utilisation selon la revendication 2, dans laquelle le cancer est sélectionné parmi le groupe constitué de : vessie, cerveau, sein, cervical, colo-rectal, oesophagien, rein, foie, poumon, naso-pharyngien, pancréatique, prostate, peau, estomac, utérin, ovarien, testiculaire, hématologique, un mélanome, un cancer rénal, un myélome, un cancer de la thyroïde, un lymphome, une leucémie, ou une lésion métastatique du cancer.

4. Composition pharmaceutique selon la revendication 1 pour l'utilisation dans un procédé de traitement d'une infection chez un sujet en ayant besoin, le procédé comprenant l'administration, au sujet, d'une quantité efficace de la composition pour favoriser ou provoquer une activation des cellules T chez le sujet.

5. Composition pharmaceutique pour l'utilisation selon la revendication 4, dans laquelle l'infection est une infection microbienne, optionnellement dans laquelle l'infection est bactérienne, fongique, ou virale.

6. Composition pharmaceutique pour l'utilisation selon la revendication 4, dans laquelle l'infection est parasite.

7. Composition pharmaceutique pour l'utilisation selon la revendication 2, dans laquelle la composition est administrée conjointement avec un vaccin, ou sous forme de composant de ce dernier.

8. Composition pharmaceutique pour l'utilisation selon la revendication 2, dans laquelle la composition est administrée sous forme de traitement adjuvant conjointement avec un traitement primaire contre le cancer.

9. Composition pharmaceutique pour l'utilisation selon la revendication 2, dans laquelle la composition est administrée sous forme de traitement adjuvant suivant l'administration d'un premier agent thérapeutique,
optionnellement dans laquelle la composition est administrée au sujet 1, 2, 3, 4, 5, 6, 7, 8, 9, 10, 11, 12, 13, ou 14 jours après l'administration du premier agent thérapeutique.

10. Composition pharmaceutique pour l'utilisation selon l'une quelconque des revendications 2 à 9, dans laquelle la composition est administrée au sujet systémiquement.

11. Composition pharmaceutique pour l'utilisation selon l'une quelconque des revendications 2 à 9, dans laquelle la composition est administrée au sujet localement à un site destiné à être traité.
